(19) Europäisches Patentamt
European Patent Office
Office européen des brevets

(11) Publication number: **0 034 798**
**B1**

(12) **EUROPEAN PATENT SPECIFICATION**

(45) Date of publication of patent specification: **22.05.85**

(51) Int. Cl.[4]: **C 07 D 207/36,**
C 07 D 401/04, A 61 K 31/40

(21) Application number: **81101133.7**

(22) Date of filing: **17.02.81**

(54) **Antiinflammatory 4,5-diaryl-2-(substituted-thio)pyrroles and their corresponding sulfoxides and sulfones.**

(30) Priority: **19.02.80 US 122501**

(43) Date of publication of application:
**02.09.81 Bulletin 81/35**

(45) Publication of the grant of the patent:
**22.05.85 Bulletin 85/21**

(84) Designated Contracting States:
**BE CH DE FR GB IT LI LU NL SE**

(56) References cited:
**EP-A-0 005 156**
**US-A-3 709 906**

**CHEMICAL ABSTRACTS, vol. 89, no. 57, July 31, 1978, page 589, abstract 42980n Columbus, Ohio, USA K.E. TEO et al. "Pyrrole chemistry, XVIII. The thio-Claisen rearrangement"**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Cherkofsky, Saul Carl**
**1013 Woodstream Drive Ramblewood**
**Wilmington Delaware 19810 (US)**

(74) Representative: **von Kreisler, Alek, Dipl.-Chem. et al**
**Deichmannhaus am Hauptbahnhof**
**D-5000 Köln 1 (DE)**

**EP 0 034 798 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

This invention relates to antiinflammatory pyrroles.

J. Szmuszkovicz et al. *J. Med. Chem., 9* (4), 527—36 (1966) described synthesis and biological activity of a clinically tested antiinflammatory agent of the formula

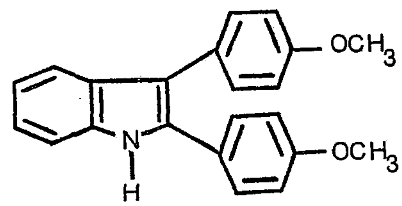

Yoshida et al. U.S.P. 3,709,906 disclose 2-alkyl-4,5-diphenylpyrrole derivatives which are useful as antiinflammatory agents.

There is a continuing need for safe and effective antiinflammatory agents. Inflammation is a disease process characterized by redness, fever, swelling, and pain. Arthritis, in its various forms, is the most prevalent, chronic, and severe of the inflammatory diseases. Traumatic injury and infection also involve inflammation, and antiinflammatory drugs are often used in their treatment. The usefulness of most commercial antiinflammatories is limited because of toxicity and adverse side-effects. Many produce gastric irritation and other effects, such as changes in blood cells and central nervous system. Adrenocortical steroids produce gastric irritation and suppression of normal adrenal function.

Summary of the Invention

This invention relates to compounds of formula I, pharmaceutical compositions containing them and methods of use of these compounds to treat arthritis or alleviate pain in mammals.

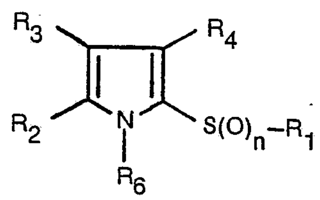

(I)

where

$R_1$ = $C_1$—$C_4$ alkyl, $C_1$—$C_4$ mono- or polyfluoroalkyl or allyl;

$R_2$ and $R_3$, independently, = 2-thienyl, 3-pyridyl 3-pyridyl-N → oxide or

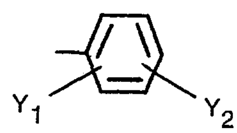

;

$Y_1$ = $C_1$—$C_4$ alkyl, $C_1$—$C_4$ alkoxy, F, Cl, Br, H, $(R_5)_2N$ or $R_5S(O)_m$ wherein $R_5$ = methyl or ethyl and m = 0, 1 or 2;

$Y_2$ = H, F or Cl;

$R_4$ = H or $C_1$—$C_3$ alkyl;

$R_6$ = H, $C_1$—$C_4$ alkyl, allyl, —$CH_2CH_2N(R_7)_2$,

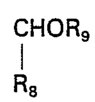

2-tetrahydropyranyl, 2-tetrahydrofuranyl,

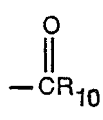

,

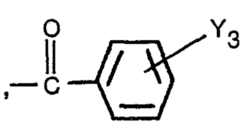

,

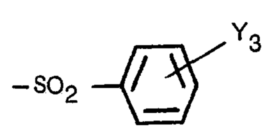

.

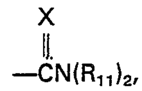

$C_1$—$C_4$ alkylsulfonyl, or

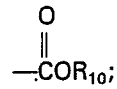

$R_7$ = H, methyl or ethyl;
$R_8$ = H or methyl;
$R_9$ = $C_1$—$C_3$ alkyl, benzyl, —$CH_2CH_2OCH_3$, or

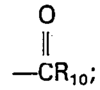

$R_{10}$ = $C_1$—$C_4$ alkyl or benzyl;
$R_{11}$ = methyl or ethyl;
X = O or S;
$Y_3$ = H, F, Cl, Br, $C_1$—$C_4$ alkyl, $C_1$—$C_4$ alkoxy or nitro;
n = 0, 1 or 2;

provided that at least one of the following must be present: at least one of $R_2$ and $R_3$ must be

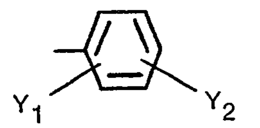 ;

where $Y_1$ = $R_5S(O)_m$ where $R_5$ is methyl or ethyl, m = 0, 1 or 2 and $Y_2$ = H, F or Cl; or its pharmaceutically suitable acid addition salt where at least one or $R_2$ or $R_3$ = 3-pyridyl, $Y_1$ = $(R_5)_2N$, or $R_6$ = —$CH_2CH_2N(R_7)_2$.

## Detailed Description of the Invention

### Preferred Compounds

Compounds preferred for their degree of activity, safety and/or ease of synthesis include those where independently:

a) $R_1$ = methyl or trifluoromethyl; or
b) $R_1$ = methyl and n = 2; or
c) $R_1$ = trifluoromethyl and n = 2; or
d) $R_2$ and $R_3$, independently are

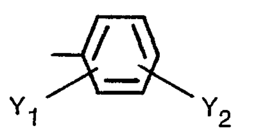 ;

and at least one of $R_2$ and $R_3$ contains $Y_1$ = $CH_3S(O)_m$; and the other of $R_2$ and $R_3$ contains $Y_1$ = F, Cl, methoxy and more preferably F; $Y_2$ = H; or

e) $R_2$ = 3-pyridyl; or
f) $R_4$ = H; or
g) $R_6$ = H; or
h) n = 0 or 2 and more preferably n = 2.

Compounds of a preferred scope include those where:
$R_1$ = methyl or trifluoromethyl;
$R_2$ and $R_3$ are

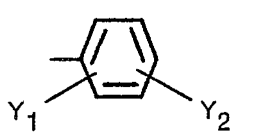 ;

and at least one of $R_2$ and $R_3$ must contain;

$Y_1 = CH_3S(O)_m$;

and the other $R_2$ and $R_3$ contain:

$Y_1$ = F, Cl or methoxy and more preferably F; $Y_2$ = H;

$R_4$ = H;

$R_6$ = H; and

n = 0 or 2 and more preferably n = 2.

Examples of compounds preferred for their activity are

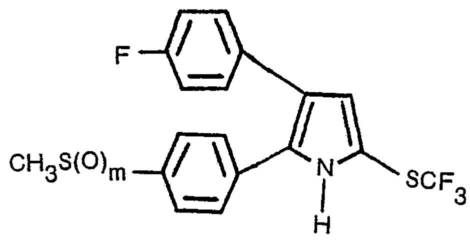

where

m = 0; m = 2.

Synthesis

The compounds of this invention can be prepared from 2,3-diarylpyrroles. One method of preparation of 2,3-diarylpyrroles involves reaction of substituted α-aminodeoxybenzoins with acetylene diesters, followed by hydrolysis and decarboxylation according to the procedure used by J. Szmuszkovicz et al, *J. Med. Chem., 9,* 527 (1966) and U.S. Patent 3,462,451 for the synthesis of 2,3-bis(4-methoxyphenyl)pyrrole. (Scheme I).

SCHEME I

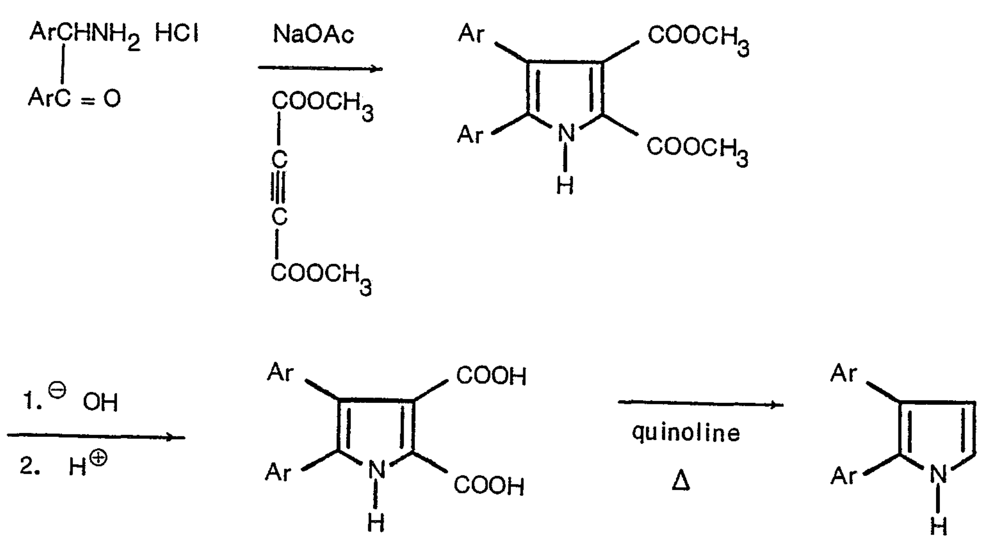


Another method of preparation of 2,3-diarylpyrroles is a modification of the procedure of T. Severin and H. Poehlmann, *Chem. Ber., 110,* 491 (1977), which describes the preparation of monoaryl pyrroles. By using substituted desoxybenzoins, the desired 2,3-diarylpyrroles result (Scheme II).

SCHEME II

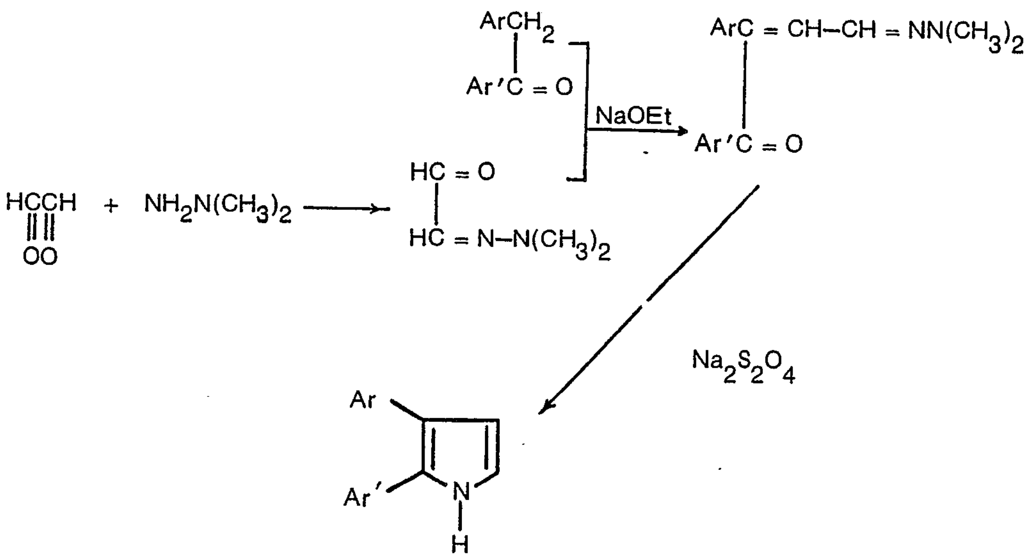


Preparation of 4,5-diaryl-3-alkylpyrroles can be accomplished by several methods. First, 4,5-diarylpyrrole-3-carboxylate esters, prepared, for instance, by the method of A. M. van Leusen et al., *Tet. Letters,* 5337 (1972) can be reduced to the 4,5-diaryl-3-methylpyrroles by lithium aluminum hydride [following the general procedure of R. L. Hinman and S. Theodoropulos, *J. Org. Chem., 28,* 3052 (1963)].

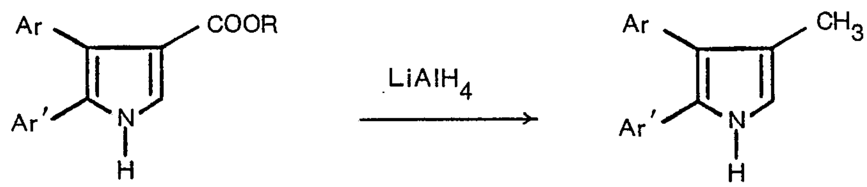


Secondly, 4,5-diaryl-3-propylpyrroles can be prepared by the thio-Claisen rearrangement of 2-allylthiopyrroles, followed by Raney nickel reduction [general procedure of K. Teo et al., *Can. J. Chem., 56,* 221 (1978)].

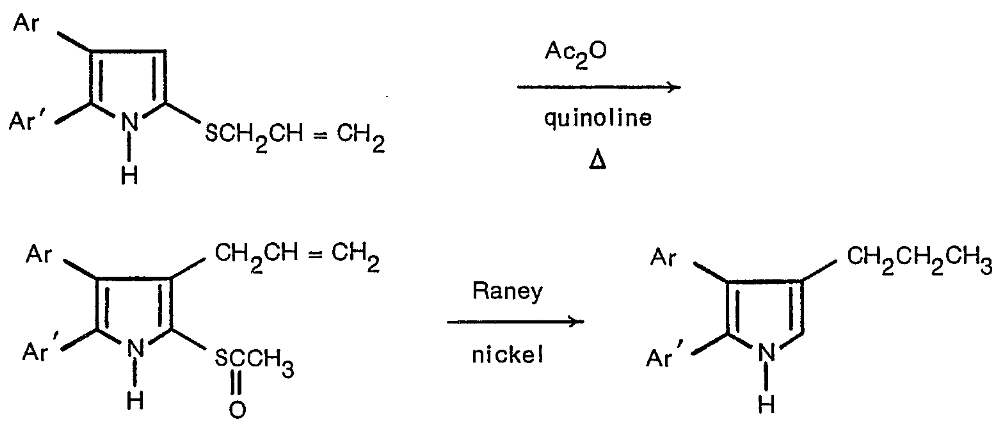


Thirdly, 4,5-diaryl-3-alkylpyrroles can be prepared by the general procedure of N. Engel and, W. Steglich, *Angew. Chem. Int. Ed. Engl., 17,* 676 (1978), from N-allylcarboxamides.

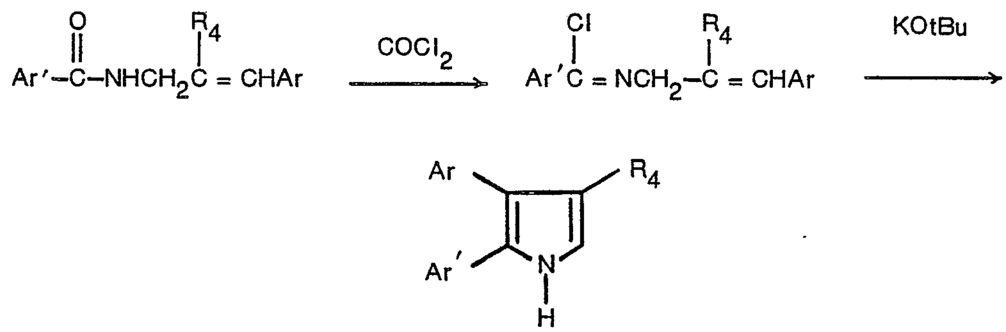


Introduction of an alkylthio functionality is accomplished in several ways. First, trifluoromethanesulfenyl chloride reacts directly and nearly quantitatively with diarylpyrroles to give the 2-trifluoromethylthio substituted compounds.

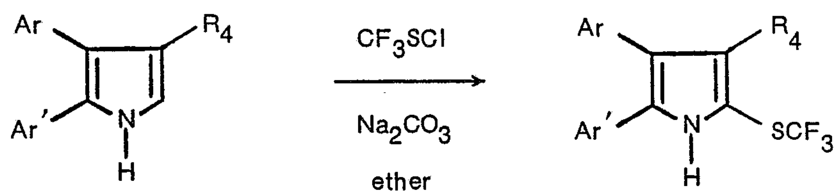


Secondly, other alkylthio pyrroles can also be prepared directly by the reaction of an alkanesulfenyl chloride (such as methanesulfenyl chloride) with a 2,3-diarylpyrrole in an inert organic solvent, such as tetrahydrofuran, diethyl ether, or dioxane, at temperatures from −80°C to 25°C.

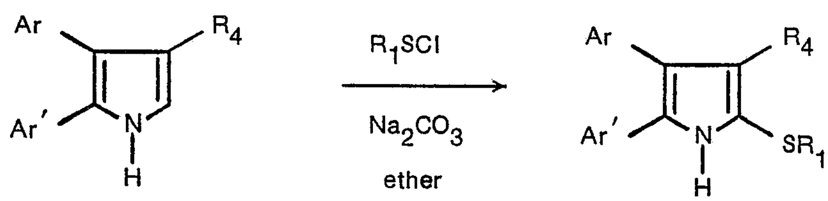


Thirdly, introduction of other alkylthio groups is also accomplished in a two step procedure involving substitution by cupric thiocyanate, thiocyanogen or the like to give the 2-thiocyanate derivative, then hydrolysis in the presence of an alkyl halide, e.g., methyl iodide, or tetrafluoroethylene.

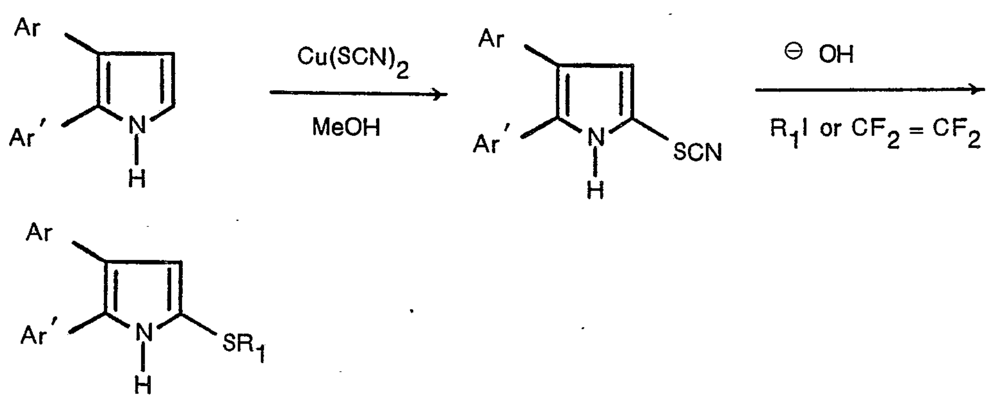

Fourthly, trifluoromethanesulfenyl chloride reacts with 4,5-diarylpyrrole-2-carboxaldehydes and 4,5-diarylpyrrole-2-carboxylic acids to give 4,5-diaryl-2-(trifluoromethylthio)pyrroles directly (with concomitant loss of carbon monoxide and carbon dioxide respectively).

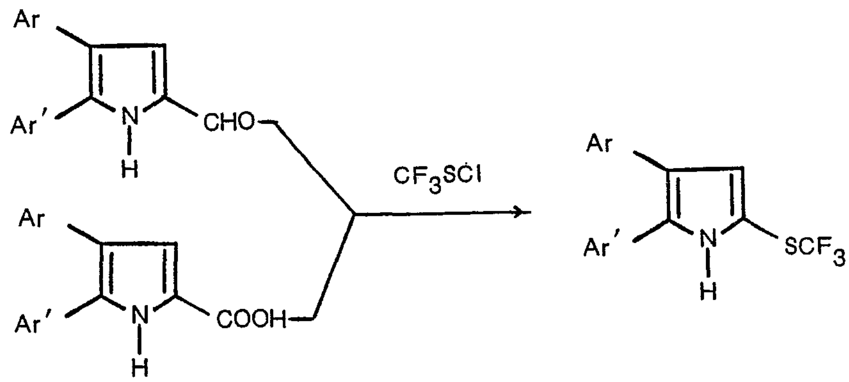


Oxidation of the 2-alkylthio compounds with suitable oxidizing agents, such as *m*-chloroperoxybenzoic acid (mClPBA) gives the corresponding sulfoxides and sulfones.

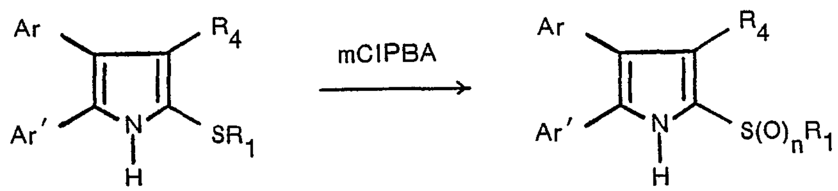


(n = 1,2)

The $R_6$-substituent other than hydrogen of formula I can be introduced by direct alkylation, acylation, or sulfonylation of the compounds of formula I where $R_6$ = H. This reaction can be carried out in the absence or presence of a base, such as potassium carbonate, pyridine, triethylamine, potassium t-butoxide, methyl lithium, dimsyl sodium or the like. The reaction can be run neat, using the reagent as solvent, or in the presence of an inert solvent, including but not limited to dimethylformamide, glyme, THF, pyridine and methylene chloride. The temperature of the reaction can be in the range −78°C to the boiling point of the solvent or reagent, if used in excess as the solvent. Examples of alkylating, acylating and sulfonylating agents that can be employed are allyl halides; alkyl halides such as methyl iodide; dimethylaminoethyl chloride; alkoxymethyl halides, such as benzyloxymethyl chloride; acyloxymethyl halides, such as chloromethylpivalate; dihydropyran; ethyl vinyl ether; 2-chlorotetrahydrofuran; alkyl chloroformates, such as ethyl chloroformate; dialkylcarbamoyl chlorides, such as diethylcarbamoyl chloride; dialkylthiocarbamoyl chlorides such as diethylthiocarbamoyl chloride; alkanoic anhydrides and alkanoyl halides, such as acetic anhydride; aroyl halides, such as benzoyl chloride; alkanesulfonyl halides such as methanesulfonyl chloride; arylsulfonyl halides, such as benzenesulfonyl chloride.

Preparation of pharmaceutically suitable salts of compounds of formula I can be in accordance with well-known techniques of forming salts.

The preparation of these compounds is further illustrated by the following Examples. All parts are by weight and temperatures are in degrees centrigrade unless otherwise specified.

## Example 1

### 3-(4-Fluorophenyl)-2-(4-methylthiophenyl)-5-(trifluoromethylthio)-1H-pyrrole

A. 2-(4-Fluorophenyl)-1-(4-methylthiophenyl)ethanone

To a stirred mixture of 75.4 g (0.438 mole) of 4-fluorophenylacetyl chloride and 54.4 g (0.438 mole) of thioanisole in 500 ml methylene chloride, chilled in an ice bath, was added in portions over approximately twenty minutes a quantity of 58.3 g (0.438 mile) of aluminum chloride. The resulting mixture was stirred in an ice bath another 1.5 hours, then was poured into 1 liter of 1N hydrochloride acid. The mixture was extracted with methylene chloride three times. The combined organic layers were washed successively with saturated aqueous sodium bicarbonate, 5% sodium hydroxide solution, saturated salt solution then dried and concentrated by rotary evaporation. The residue was triturated with cold ethanol and collected to give 99.7 g (88%) of off-white solid, m.p. 135—138°.

B. 2-(4-Fluorophenyl-4-(dimethylhydrazono)-1-(4-methylthiophenyl)-2-buten-1-one

To a mixture of 52 g (0.2 mole) of 2-(4-fluorophenyl)-1-(4-methylthiophenyl)ethanone and 22 g (0.22 mole) of glyoxal mono(dimethylhydrazone) [prepared by the method of T. Severin and H. Poehlmann, *Chem. Ber., 110,* 491 (1977)] in 200 ml ethanol was added dropwise a solution of sodium ethoxide prepared from 5.1 g (0.22 mole) of sodium in 200 ml ethanol. The mixture was heated at reflux for three hours, then stirred at room temperature overnight. The mixture was concentrated by rotary evaporation. The residue was diluted with water and extracted three times with methylene chloride. The organic layers were combined, dried and concentrated. The residue was recrystallized from methyl cyclohexane to give 46.4 g (68%) of yellow solid, m.p. 129—130°.

C. 3-(4-Fluorophenyl)-2-(4-methylthiophenyl)-1H-pyrrole.

To a stirred mixture of 46.4 g (0.136 mole) of 2-(4-fluorophenyl)-4-(dimethylhydrazono)-1-(4-methylthiophenyl)-2-buten-1-one in 400 ml ethanol and 250 ml water was added all at once 153.8 g (0.884 mole) of sodium hydrosulfate. The mixture was heated at reflux for three hours, then cooled to room temperature. A quantity of 50 ml water was added, then the mixture was poured into 2 liters of ice water, the solid which precipitated was collected, washed with water, dried, then recrystallized from ethanol/water to give 28.2 g (87%) of white solid, m.p. 164—165°.

D. 3-(4-Fluorophenyl)-2-(4-methylthiophenyl)-5-(trifluoromethylthio)-1H-pyrrole

To a stirred mixture of 2.8 g (0.01 mole) of 3-(4-fluorophenyl)-2-(4-methylthiophenyl)-1H-pyrrole and 2.1 g (0.02 mole) of sodium carbonate in 50 ml ether at −78° and under a dry ice condenser was added a solution of 1.5 g (0.011 mole) of trifluoromethanesulfenyl chloride in 2 ml of cold ether. The mixture was stirred at −78° for one hour, then allowed to warm to room temperature. The mixture was filtered to remove the inorganic salts. The filtrate was concentrated and the residue was purified by chromatography on silica gel, eluting with hexane/toluene mixtures (60/40). The chromatographed product was recrystallized from ethanol/water to give 3.5 g of white solid, m.p. 106—107°.

*Anal.* Calcd. for $C_{18}H_{13}F_4NS_2$: C, 56.39; H, 3.42; N, 3.65.
Found: C, 56.30; H, 3.52; N, 3.71.

Example 2

3-(4-Fluorophenyl)-2-(4-methylsulfonylphenyl)-5-(trifluoromethylthio)-1H-pyrrole

A. 3-(4-Fluorophenyl)-2-(4-methylsulfonylphenyl)-1H-pyrrole

To a stirred mixture of 28.3 g (0.1 mole) of 3-(4-fluorophenyl)-2-(4-methylthiophenyl)-1H-pyrrole in 850 ml ethyl acetate in an ice bath was added dropwise a solution of 50 g (~0.25 mole) of ~85% m-chloroperoxybenzoic acid in 500 ml ethyl acetate. The mixture was stirred at 0° for three hours, then at room temperature overnight. The mixture was cooled back to 0° and the crystalline product was collected, washed with cold ethyl acetate, then dried. F-NMR indicated the solid contained 80% sulfone and ~20% sulfoxide, so the solid was dissolved in 2 liters of hot ethyl acetate (at 65°) and a quantity of 4.0 g of m-chloroperoxybenzoic acid was added. The mixture was allowed to cool to room temperature, then chilled in an ice bath. The crystalline precipitate was collected, washed with cold ethyl acetate, then air dried to give 17.1 g (54%) of off-white product; m.p. 268—270°.

B. 3-(4-Fluorophenyl)-2-(4-methylsulfonylphenyl)-5-(trifluoromethylthio)-1H-pyrrole.

To a mixture of 15.8 g (0.05 mole) of 3-(4-fluorophenyl)-2-(4-methylsulfonylphenyl)-1H-pyrrole and 10.6 g (0.1 mole) of sodium carbonate in 300 ml tetrahydrofuran and 300 ml ether at −78° and under a dry ice condenser was added as a gas ~12.8 g (0.095 mole) of trifluoromethanesulfenyl chloride. The mixture was allowed to warm to room temperature (with the dry ice condenser still attached) and stirred for three hours. The mixture was chilled again to −78° and another ~3.0 g of trifluoromethanesulfenyl chloride was added as a gas. The mixture was stirred overnight at room temperature (with dry ice in the condenser for several hours). Another 3.0 g trifluoromethanesulfenyl chloride was added as above and the mixture was stirred for three days at room temperature. The mixture was filtered to remove the inorganics and the filtrate was concentrated to give 21.8 g of solid. This was purified by chromatography on silica gel, eluting with toluene containing 4 to 8% ethyl acetate to give, after recrystallization from ethanol/water, 18.2 g (88%) of product as a white solid, m.p. 204—206°. A previous sample prepared by a similar procedure had m.p. 203—204° and gave the microanalytical results shown below.

*Anal.* Calcd. for $C_{18}H_{13}F_4NO_2S_2$: C, 52.04; H, 3.15; N, 3.37.
Found: C, 52.29; H, 3.17; N, 3.25.

Dosage Forms

The anti-arthritic agents of this invention can be administered to treat arthritis by any means that produces contact of the active agent with the agent's site of action in the body of a mammal. They can be administered by any conventional means available for use in conjunction with pharmaceuticals; either as individual therapeutic agents or in a combination of therapeutic agents. They can be administered alone, but are generally administered with a pharmaceutical carrier selected on the basis of the chosen route of administration and standard pharmaceutical practice.

The dosage administered will, of course, vary depending upon known factors such as the pharmacodynamic characteristics of the particular agent, and its mode and route of administration; age, health, and weight of the recipient; nature and extent of symptoms, kind of concurrent treatment, frequency of treatment, and the effect desired. Usually a daily dosage of active ingredient can be about 0.05 to 40 milligrams per kilogram of body weight. Ordinarily 0.1 to 20, and preferably 0.2 to 10 milligrams per kilogram per day given in divided doses 2 to 4 times a day or in sustained release form is effective to obtain desired results.

Dosage forms (compositions) suitable for internal administration contain from about 5 milligrams to about 500 milligrams of active ingredient per unit. In these pharmaceutical compositions the active ingredient will ordinarily be present in an amount of about 0.5—95% by weight based on the total weight of the composition.

The active ingredient can be administered orally in solid dosage forms, such as capsules, tablets, and powders, or in liquid dosage forms, such as elixirs, syrups, and suspensions, it can also be administered parenterally, in sterile liquid dosage forms.

Gelatin capsules contain the active ingredient and powdered carriers, such as lactose, sucrose, mannitol, starch, cellulose derivatives, magnesium stearate, stearic acid, and the like. Similar diluents can be used to make compressed tables. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration contain preferably a water soluble salt of the active ingredient, suitable stabilizing agents, and if necessary, buffer substances. Antioxidizing agents such as sodium bisulfite, sodium sulfite, or ascorbic acid either alone or combined are suitable stabilizing agents. Also used are citric acid and its salts and sodium EDTA. In addition parenteral solutions can contain preservatives, such as benzalkonium chloride, methyl- or propyl-paraben, and chlorobutanol.

Suitable pharmaceutical carriers are described in *Remington's Pharmaceutical Sciences*, E. W. Martin, a standard reference text in this field.

Useful pharmaceutical dosage-forms for administration of the compounds of this invention can be illustrated as follows:

### Capsules

A large number of unit capsules are prepared by filling standard two-piece hard gelatin capsules each with 50 milligrams of powdered active ingredient, 110 milligrams of lactose, 32 milligrams of talc, and 8 milligrams magnesium stearate.

### Capsules

A mixture of active ingredient in soybean oil is prepared and injected by means of a positive displacement pump into gelatin to form soft gelatin capsules containing 50 milligrams of the active ingredient. The capsules are washed in petroleum ether and dried.

### Tablets

A large number of tablets are prepared by conventional procedures so that the dosage unit is 50 milligrams of active ingredient, 7 milligrams of ethyl cellulose, 0.2 milligrams of colloidal silicon dioxide, 7 milligrams of magnesium stearate, 11 milligrams of microcrystalline cellulose, 11 milligrams of cornstarch and 98.8 milligrams of lactose. Appropriate coatings may be applied to increase palatability or delay absorption.

### Pharmaceutical Utility

A procedure for detecting and comparing the anti-inflammatory activity of compounds in this series and standard drugs for which there is a good correlation with human efficacy is the adjuvant-induced arthritis test in rats.

A procedure for detecting and comparing the analgesic activity of compounds in this series and standard drugs for which there is a good correlation with human efficacy is the phenylquinone writhing test.

The test procedures employed for determining antiinflammatory activity are described below with test data included in Table I.

### Established Adjuvant-Induced Arthritis in Rats

Charles River Lewis male rats (130—150 grams) are injected subcutaneously in plantar area of the right hind paw with 0.1 ml of adjuvant (Difco heat-killed, lyophilized *Mycobacterium butyricum* suspended in mineral oil 5 mg/ml). 20 Non-arthritic controls are injected with mineral oil. The animals are held for 2 weeks to allow development of arthritis. Paw volumes (uninjected, left hind paw) are measured and the

adjuvant injected rats are culled and distributed to treatment groups of 10 of equal disease severity. Non-arthritic controls are distributed to 2 groups of 10. The rats are given oral doses of compound or PVA-Acacia (Polyvinyl Alcohol 1%, Gum Acacia, U.S.P. 5%, Methylparaben 0.5%) (10 ml/kg) by gavage on that day and on the 6 following days. One day after the last dose the paw volumes (uninjected, left hind paw) are measured using a Ugo Basile Volume Differential Meter Model 7101.

$$\frac{\text{Arthritic Control Mean Paw Volume (ml)} - \text{Treatment Group Mean Paw Volume (ml)}}{\text{Arthritic Control Mean Paw Volume (ml)} - \text{Non-Arthritic Control Mean Paw Volume (ml)}} \times 100 = \begin{matrix}\text{\% Decrease from}\\ \text{Control Mean}\\ \text{Paw Volume}\end{matrix}$$

Dose-response regression lines of the % decrease are plotted on semi-log paper by visual fit and the ED50% decrease from control paw volume is determined by inspection.

TABLE I

Biological Activity

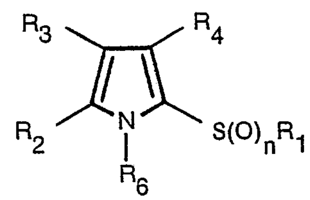

| Example | $R_3$ | $R_2$ | $R_1$ | n | $R_4$ | $R_6$ | Adjuvant Arthritis $ED_{50}$ (mg/kg) |
|---|---|---|---|---|---|---|---|
| 1 | $4\text{-}FC_6H_4$ | $4\text{-}CH_3SC_6H_4$ | $CF_3$ | O | H | H | 0.6 |
| 2 | $4\text{-}FC_6H_4$ | $4\text{-}CH_3SO_2C_6H_4$ | $CF_3$ | O | H | H | 0.15 |

**Claims**

1. A compound of the formula

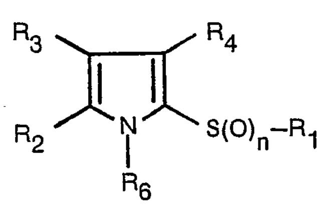

(I)

where

$R_1$ = $C_1$—$C_4$ alkyl, $C_1$—$C_4$ mono- or polyfluoroalkyl or allyl;

$R_2$ and $R_3$, independently, = 2-thienyl, 3-pyridyl 3-pyridyl-N-oxide or

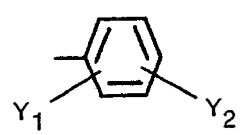

;

$Y_1$ = $C_1$—$C_4$ alkyl, $C_1$—$C_4$ alkoxy, F, Cl, Br, H, $(R_5)_2N$ or $R_5S(O)_m$ wherein $R_5$ = methyl or ethyl and m = 0, 1 or 2;

$Y_2$ = H, F or Cl;
$R_4$ = H or $C_1$—$C_3$ alkyl;
$R_6$ = H, $C_1$—$C_4$ alkyl, allyl, —$CH_2CH_2N(R_7)_2$,

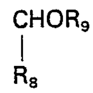

2-tetrahydropyranyl, 2-tetrahydrofuranyl,

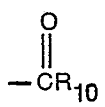, 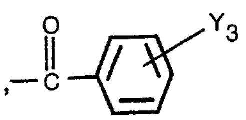, 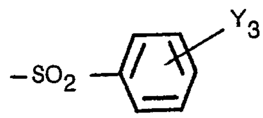

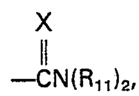

$C_1$—$C_4$ alkylsulfonyl, or

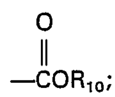

$R_7$ = H, methyl or ethyl;
$R_8$ = H or methyl;
$R_9$ = $C_1$—$C_3$ alkyl, benzyl, —$CH_2CH_2OCH_3$, or

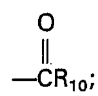

$R_{10}$ = $C_1$—$C_4$ alkyl or benzyl;
$R_{11}$ = methyl or ethyl;
X = O or S;
$Y_3$ = H, F, Cl, Br, $C_1$—$C_4$ alkyl, $C_1$—$C_4$ alkoxy or nitro; and
n = 0, 1 or 2;

provided that at least one of the following must be present: at least one of $R_2$ and $R_3$ must be

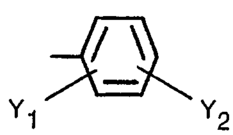 ;

where $Y_1$ = $R_5S(O)_m$ where $R_5$ is methyl or ethyl, m = 0, 1 or 2 and $Y_2$ = H, F or Cl; or its pharmaceutically suitable acid addition salt where at least one or $R_2$ or $R_3$ = 3-pyridyl, $Y_1$ = $(R_5)_2N$, or $R_6$ = —$CH_2CH_2N(R_7)_2$.

2. A compound of Claim 1 where $R_1$ = methyl or trifluoromethyl.

3. A compound of Claim 2 where $R_1$ = methyl and n = 2.

4. A compound of Claim 2 where $R_1$ = trifluoromethyl and n = 2.

5. A compound of Claim 1 where $R_2$ and $R_3$, independently are

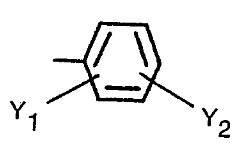 ;

and at least one of $R_2$ and $R_3$ contains $Y_1$ = $CH_3S(O)_m$ and the other of $R_2$ and $R_3$ contains $Y_1$ = F, Cl or methoxy.

6. A compound of Claim 5 where $Y_1$ = F.

7. A compound of Claim 5 where $Y_2$ = H.
8. A compound of Claim 1 where $R_2$ = 3-pyridyl.
9. A compound of Claim 1 where $R_4$ = H.
10. A compound of Claim 1 wherein $R_6$ = H.
11. A compound of Claim 1 where n = 0 or 2.
12. A compound of Claim 1 where n = 2.
13. A compound of Claim 1 where $R_1$ = methyl or trifluoromethyl; $R_2$ and $R_3$ are

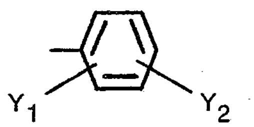

;

and at least one of $R_2$ and $R_3$ contains $Y_1 = CH_3S(O)_m$; and the other $R_2$ and $R_3$ contains $Y_1$ = F, Cl or methoxy; $Y_2$ = H; $R_4$ = H; $R_6$ – H; and n = 0 or 2.

14. A compound of Claim 1 of the formula:

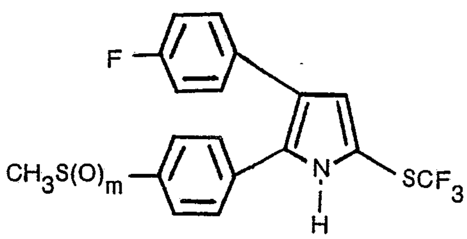

where m = 0 or 2.

15. The compound of Claim 14 where m = 2.

16. A pharmaceutical composition consisting essentially of a suitable pharmaceutical carrier and an effective antiinflammatory amount of a compound of any of Claims 1—15.

17. A process for preparing a compound of Claim 1 which comprises:
(a) contacting a compound of the formula

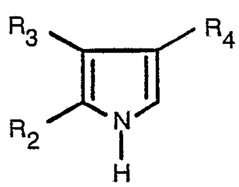

where $R_2$, $R_3$ and $R_4$ are as defined in claim 1 with a sulfenyl chloride, $R_1SC1$ where $R_1$ is as defined in claim 1; and optionally,
(b) contacting the product of step (a) with a suitable oxidizing agent;
(c) optionally alkylating, acylating or sulfonylating the product of (a) or (b); and
(d) optionally converting the product of (a), (b) or (c) into a pharmaceutically suitable salt.

18. A process for preparing a compound of Claim 1 which comprises:
(a) contacting a compound of the formula

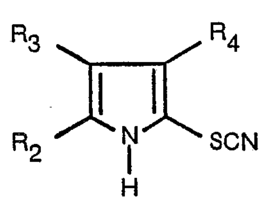

where $R_2$, $R_3$ and $R_4$ are as defined in claim 1 with an alkyl halide, $R_1X$, where $R_1$ is as defined in claim 1, or tetrafluoro-ethylene, in the presence of suitable inorganic base; and, optionally,
(b) contacting the product of step (a) with a suitable oxidizing agent;
(c) optionally alkylating, acylating or sulfonylating the product of (a) or (b); and
(d) optionally converting the product of (a), (b) or (c) into a pharmaceutically suitable salt.

**Patentansprüche**

1. Verbindung der Formel

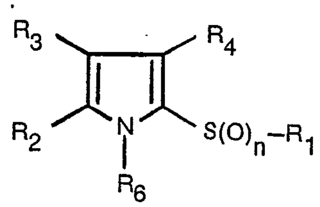

(I)

in der

$R_1$ = $C_1$- bis $C_4$-Alkyl, $C_1$- bis $C_4$-Mono- oder Polyfluorolkyl oder Allyl,

$R_2$ und $R_3$ unabhängig voneinander = 2-Thienyl, 3-Pyridyl, 3-Pyridyl-N-oxid oder

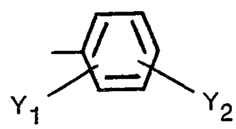

;

$Y_1$ = $C_1$- bis $C_4$-Alkyl, $C_1$- bis $C_4$-Alkoxy, F, Cl, Br, H, $(R_5)_2N$ oder $R_5S(O)_m$, worin $R_5$ = Methyl oder Ethyl und m = 0, 1, oder 2,

$Y_2$ = H, F oder Cl,

$R_4$ = H oder $C_1$- bis $C_3$-Alkyl,

$R_6$ = H, $C_1$- bis $C_4$-Alkyl, Allyl, $-CH_2CH_2N(R_7)_2$,

$$-\underset{R_8}{\underset{|}{C}}HOR_9,$$

2-Tetrahydropyranyl, 2-Tetrahydrofuranyl,

$-\overset{O}{\overset{\|}{C}}R_{10}$, 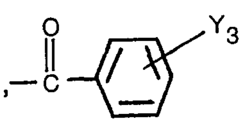 , 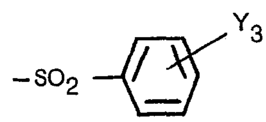

$$-\overset{X}{\overset{\|}{C}}N(R_{11})_2,$$

$C_1$- bis $C_4$-Alkylsulfonyl oder

$$-\overset{O}{\overset{\|}{C}}OR_{10},$$

$R_7$ = H, Methyl oder Ethyl,

$R_8$ = H oder Methyl,

$R_9$ = $C_1$-bis $C_3$-Alkyl, Benzyl, $-CH_2CH_2OCH_3$ oder

$$-\overset{O}{\overset{\|}{C}}R_{10},$$

$R_{10}$ = $C_1$- bsi $C_4$-Alkyl oder Benzyl,

$R_{11}$ = Methyl oder Ethyl,

X = O oder S,

$Y_3$ = H, F, Cl, Br, $C_1$- bis $C_4$-Alkyl, $C_1$- bis $C_4$-Alkoxy oder Nitro und

n = 0, 1 oder 2,

mit der Maßgabe, daß wenigstens einer der folgenden Substituenten vorhanden sein muß:

wenigstens einer der Substituenten $R_2$ und $R_3$ muß

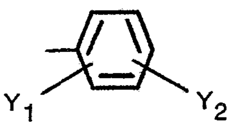

;

sein, worin

$Y_1 = R_5S(O)_m$, worin $R_5$ Methyl oder Ethyl ist und m = 0, 1 oder 2, und

$Y_2$ = H, F oder Cl,

oder ihr pharmazeutisch geeignetes Säureadditionssalz, in dem wenigstens einer der Substituenten $R_2$ und $R_3$ = 3-Pyridyl, $Y_1 = (R_5)_2N$ oder $R_6 = -CH_2CH_2N(R_7)_2$.

2. Verbindung nach Anspruch 1, dadurch gekennzeichnet, daß $R_1$ = Methyl oder Trifluoromethyl.

3. Verbindung nach Anspruch 2, dadurch gekennzeichnet, daß $R_1$ = Methyl und n = 2.

4. Verbindung nach Anspruch 2, dadurch gekennzeichnet, daß $R_1$ = Trifluoromethyl und n = 2.

5. Verbindung nach Anspruch 1, dadurch gekennzeichnet, daß $R_2$ und $R_3$ unabhängig voneinander

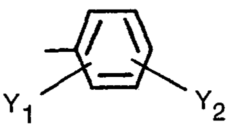

;

sind und wenigstens einer der Substituenten $R_2$ und $R_3$ $Y_1 = CH_3S(O)_m$ enthält und der andere der Substituenten $R_2$ und $R_3$ $Y_1$ = F, Cl oder Methoxy enthält.

6. Verbindung nach Anspruch 5, dadurch gekennzeichnet, daß $Y_1$ = F.

7. Verbindung nach Anspruch 5, dadurch gekennzeichnet, daß $Y_2$ = H.

8. Verbindung nach Anspruch 1, dadurch gekennzeichnet, daß $R_2$ = 3-Pyridyl.

9. Verbindung nach Anspruch 1, dadurch gekennzeichnet, daß $R_4$ = H.

10. Verbindung nach Anspruch 1, dadurch gekennzeichnet, daß $R_6$ = H.

11. Verbindung nach Anspruch 1, dadurch gekennzeichnet, daß n = 0 oder 2.

12. Verbindung nach Anspruch 1, dadurch gekennzeichnet, daß n = 2.

13. Verbindung nach Anspruch 1, dadurch gekennzeichnet, daß $R_1$ = Methyl oder Trifluoromethyl, $R_2$ und $R_3$

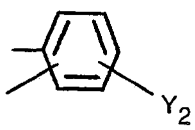

;

sind und wenigstens einer der Substituenten $R_2$ und $R_3$, $Y_1 = CH_3S(O)_m$ enthält und der andere der Substituenten $R_2$ und $R_3$,

$Y_1$ = F, Cl oder Methoxy enthält. $Y_2$ = H, $R_4$ = H, $R_6$ = H und n = 0 oder 2.

14. Verbindung nach Anspruch 1 mit der Formel

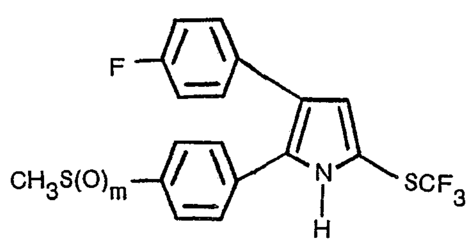

in der m = 0 oder 2.

15. Verbindung nach Anspruch 14, dadurch gekennzeichnet, daß m = 2.

16. Pharmazeutische Zusammensetzung, bestehend im wesentlichen aus einem geeigneten pharmazeutischen Träger und einer wirksamen antiinflammatorischen Menge einer Verbindung nach irgendeinem der Ansprüche 1 bis 15.

17. Verfahren zur Herstellung einer Verbindung nach Anspruch 1, dadurch gekennzeichnet, daß

(a) eine Verbindung der Formel

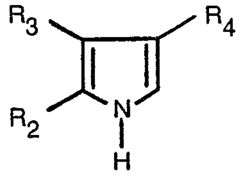

in der $R_2$, $R_3$ und $R_4$ die in Anspruch 1 angegebenen Bedeutungen haben, mit einem Sulfenylchlorid $R_1SCl$, in dem $R_1$ die in Anspruch 1 angegebene Bedeutung hat, in Berührung gebracht wird und gegebenenfalls.

(b) das Produkt aus Schritt (a) mit einem geeigneten Oxidationsmittel in Berührung gebracht wird,

(c) das Produkt aus (a) oder (b) gegebenenfalls alkyliert, acyliert oder sulfonyliert wird und

(d) gegebenenfalls des Produkt aus (a), (b) oder (c) in ein pharmazeutisch geeignetes Salz umgewandelt wird.

18. Verfahren zur Herstellung einer Verbindung nach Anspruch 1, dadurch gekennzeichnet, daß

(a) eine Verbindung der Formel

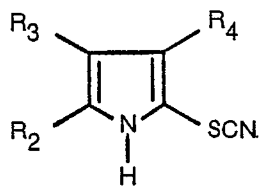

in der $R_2$, $R_3$ und $R_4$ die in Anspruch 1 angegebenen Bedeutungen haben, mit einem Alkylhalogenid $R_1X$, in dem $R_1$ die in Anspruch 1 angegebene Bedeutung hat, oder Tetrafluoroethylen in Gegenwart einer geeigneten anorganischen Base in Berührung gebracht wird und gegebenenfalls

(b) das Produkt aus Schritt (a) mit einem geeigneten Oxidationsmittel in Berührung gebracht wird,

(c) das Produkt aus (a) oder (b) gegebenenfalls alkyliert, acyliert oder sulfonyliert wird und

(d) gegebenenfalls des Produkt aus (a), (b) oder (c) in ein pharmazeutisch geeignetes Salz umgewandelt wird.

**Revendications**

1. Un composé de la formule:

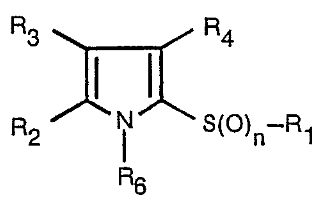

(I)

où

$R_1$ = alcoyle en $C_1$—$C_4$, mono- ou polyfluoroalcoyle en $C_1$—$C_4$ ou allyle;

$R_2$ et $R_3$, indépendamment = 2-thiényle, 3-pyridyle, 3-pyridyl-N-oxyde ou

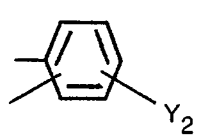

;

$Y_1$ = alcoyle en $C_1$—$C_4$, alcoxy en $C_1$—$C_4$, F, Cl, Br, H, $(R_5)_2N$ ou $R_5S(O)_m$, où $R_5$ = méthyle ou éthyle, et m = 0,1 ou 2;

$Y_2$ = H, F ou Cl;

$R_4$ = H ou alcoyle en $C_1$—$C_3$;

$R_6$ = H, alcoyle en $C_1$—$C_4$, allyle, $—CH_2CH_2N(R_7)_2$,

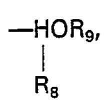

2-tétrahydropyranyle, 2-tétrahydrofuranyle,

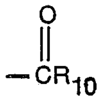, 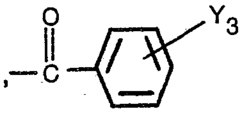, 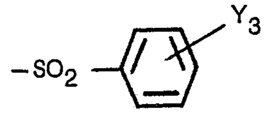

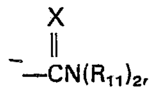,

alcoylsulfonyle en $C_1$—$C_4$, ou

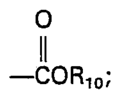;

$R_7$ = H, méthyle ou éthyle;
$R_8$ = H ou méthyle;
$R_9$ = alcoyle en $C_1$—$C_3$, benzyle, —$CH_2CH_2OCH_3$, ou

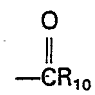

$R_{10}$ = alcoyle en $C_1$—$C_4$ ou benzyle;
$R_{11}$ = méthyle ou éthyle;
X = O ou S;
$Y_3$ = H, F, Cl, Br, alcoyle en $C_1$—$C_4$, alcoxy en $C_1$—$C_4$ ou nitro; et
n = 0,1 ou 2;
avec la condition qu'au moins l'un des suivants doit être présent:
au moins l'un de $R_2$ et $R_3$ doit être

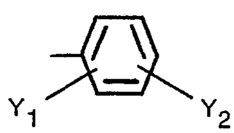;

où $Y_1$ = $R_5S(O)_m$ où $R_5$ est un radical méthyle ou éthyle m = 0, 1 ou 2 et $Y_2$ = H, F ou Cl;
ou son sel d'addition d'acide pharmaceutiquement acceptable où au moins l'un de $R_2$ et $R_3$ = 3-pyridyle, $Y_1$ = $(R_5)_2N$, ou $R_6$ = —$CH_2CH_2N(R_7)_2$.

2. Un composé selon la revendication 1, dans lequel $R_1$ = méthyle ou trifluorométhyle.
3. Un composé selon la revendication 2, dans lequel $R_1$ = methyle et n = 2.
4. Un composé selon la revendication 2, dans lequel $R_1$ = trifluorométhyle et n = 2.
5. Un composé selon la revendication 1, dans lequel $R_2$ et $R_3$ sont indépendamment

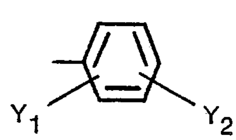;

et au moins l'un de $R_2$ et $R_3$ contient $Y_1$ = $CH_3S(O)_m$ et l'autre de $R_2$ et $R_3$ contient $Y_1$ = F, Cl ou méthoxy.
6. Un composé selon la revendication 5, dans lequel $Y_1$ = F.
7. Un composé selon la revendication 5, dans lequel $Y_2$ = H.
8. Un composé selon le revendication 1, dans lequel $R_2$ = 3-pyridyle.
9. Un composé selon la revendication 1, dans lequel $R_4$ = H.
10. Un composé selon la revendication 1, dans lequel $R_6$ = H.
11. Un composé selon la revendication 1, dans lequel n = 0 ou 2.
12. Un composé selon la revendication 1, dans lequel n = 2.
13. Un composé selon la revendication 1, dans lequel $R_1$ = méthyle ou trifluorométhyle; $R_2$ et $R_3$ sont

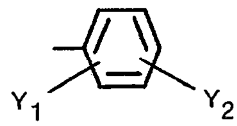

;

et au moins l'un de $R_2$ et $R_3$ contient $Y_1 = CH_3S(O)_m$; et l'autre de $R_2$ et $R_3$ contient $Y_2$ = F, Cl ou méthoxy; $Y_2$ = H; $R_4$ = H; $R_6$ = H; et n = 0 ou 2.

14. Un composé selon la revendication 1 de la formule

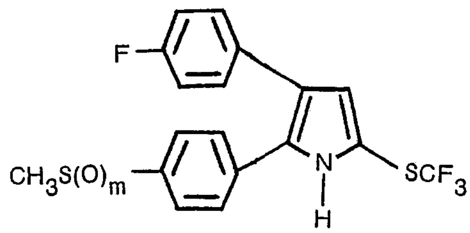

où m = 0 ou 2.

15. Le composé de la revendication 14, dans lequel m = 2.

16. Une composition pharmaceutique consituée essentiellement d'un véhicule pharmaceutique approprié et d'une quantité anti-inflammatoire efficace d'un composé selon l'une quelconque des revendications 1 à 15.

17. Un procédé de préparation d'un composé selon la revendication 1, qui comprend:

(a) la mise en contact d'un composé de la formule

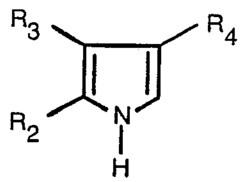

où $R_2$, $R_3$ et $R_4$ sont tels que définis à la revendication 1, avec un chlorure de sulfényle, $R_1SCl$, où $R_1$ est tel que défini à la revendication 1; et facultativement,

(b) la mise en contact du produit de l'étape (a) avec un agent oxydant approprié;

(c) facultativement l'alcoylation, l'acylation ou la sulfonylation du produit de (a) ou (b); et

(d) facultativement la conversion du produit de (a), (b) ou (c) en un sel pharmaceutiquement acceptable.

18. Un procédé de préparation d'un composé selon la revendication 1, qui comprend:

(a) la mise en contact d'un composé de la formule

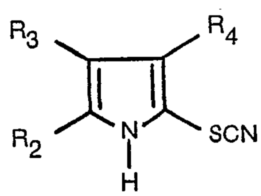

,

où $R_2$, $R_3$ et $R_4$ sont tels que définis à la revendication 1, avec un halogénure d'alcoyle, $R_1X$, où $R_1$ et tel que défini à la revendication 1, ou du tétrafluoroéthylène, en présence d'une base minérale convenable; et, facultativement

(b) la mise en contact du produit de l'étape (a) avec un agent oxydant approprié;

(c) facultativement, l'alcoylation, l'acylation ou la sulfonylation du produit de (a) ou (b); et

(d) facultativement, la conversion du produit de (a), (b) ou (c) en un sel pharmaceutiquement acceptable.